# EUROPEAN PATENT SPECIFICATION

(11) **EP 1 490 140 B1**
(45) Date of publication and mention of the grant of the patent: **25.06.2008**
(21) Application number: 03744678.8
(22) Date of filing: 14.03.2003
(51) Int. Cl.: A61L 27/54, A61L 29/16, A61L 31/16

(54) **MEDICAL DEVICES EXHIBITING ANTIBACTERIAL PROPERTIES**
MEDIZINISCHE VORRICHTUNGEN MIT ANTIBAKTERIELLEN EIGENSCHAFTEN
DISPOSITIFS MEDICAUX FAISANT PREUVE DE PROPRIETES ANTIBACTERIENNES

(30) Priority: 14.03.2002 US 364335 P
(43) Date of publication of application: 29.12.2004
(73) Proprietor: Ash Access Technology, Inc., Lafayette, Indiana 47904 (US)
(72) Inventor: ASH, Stephen, R., Lafayette, IN 47905 (US); STECZKO, Janusz, West Lafayette, IN 47906 (US)
(74) Representative: Atkinson, Peter Birch
(86) International application number: PCT/US2003/007838
(87) International publication number: WO 2003/077866

(56) References cited:
- US-A- 4 885 855
- US-A- 5 709 672
- US-A- 6 025 312
- US-B1- 6 183 764

## Description

### CROSS-REFERENCE TO RELATED APPLICATION

The present application claims the benefit of United States Provisional Application Serial No. 60/364,335 filed on March 14, 2002, which is hereby incorporated by reference in its entirety.

### BACKGROUND OF THE INVENTION

In general this invention is related to a method of manufacturing polymeric material for implantable and other medical devices exhibiting antimicrobial activity.

More specifically, the present invention is directed to implantable and other medical devices that include a polymeric component or matrix impregnated with a releasable organic dye composition exhibiting antimicrobial activity and to the preparation and use of the medical devices.

The progress of modem medicine has been advanced, in part, by the wide use of invasive medical devices, including catheters. Several million intravascular catheters are purchased each year by US hospitals and clinics. Use of these devices places large numbers of patients at risk for catheter-related bloodstream infection (2) (CRBSI). Most serious infections, such as bacteremia or fungemia, are associated with central venous catheters (CVCs) rather than small peripheral catheters (3-5). Even when aseptic techniques are used during insertion and maintenance of the catheter, at least 1 and up to 5 of every 20 CVCs inserted will be associated with an episode of bloodstream infection (6). The mortality attributable to these infections in prospective studies is 12 to 25% (5, 7), and the cost attributable for each event is between $3,700 and $29,000 (7, 8). In chronic CVCs for dialysis, the incidence of infection is 4-6% of the patient population each month (36).

Several factors pertaining to the pathogenesis of CRBSI have been identified during the last decade. Most common catheter-related bloodstream infections originate from microbes colonizing catheter hubs and the skin surrounding the insertion site (9-11). Therefore, successful preventive strategies should reduce colonization of microbes at the catheter insertion site on or about the hubs and/or minimize microbial infection toward the intravascular segment of the catheter. Inhibiting the adherence and growth of pathogens reaching the intravascular catheter segment would also be advantageous. Organisms that adhere to the catheter surface maintain themselves by producing "extracellular slime", a substance rich in exopolysaccharides, often referred to as fibrous glycocalyx or microbial biofilm (12, 13). The organisms embed themselves in the biofilm layer, becoming more resistant to the antimicrobial agents due to a dormant metabolism with different metabolic pathways than normal bacteria (14, 15). The colonization of microbes on and about CVCs is very common (16). The risk of infection is directly proportional to the quantitative level of organisms multiplying on the surface of the intravascular segment of the catheter. Several factors can potentiate the multiplication and spread of microorganisms from biofilm increasing the risk of bloodstream infections, including breaking of stalks of bacterial biofilm from the surface of the catheter. Once in the bloodstream, bacteria can multiply and cause serious illness such as sepsis (systemic inflammatory response syndrome) or metastatic infections (in bones, joints, heart valves, skin, etc.). Finally, catheter-related infection can result in local septic thrombophlebitis, having its origin in a thrombin sheath that often covers the internal and external surface of the intravascular segment of the catheter. The sheath is composed of many different proteins such as fibrin, fibrinogen, fibrinectin, laminin, thrombospondin, and collagen that strongly bind some microorganisms such as Staphylococcus aureus, Candida albicans, or coagulase-negative staphylococci. The environment is ideal for multiplication of microbes; therefore, a correlation between thrombosis and infection can be observed at the clinical level (17).

Historically, there have been four approaches for preventing catheter infection.

First, aseptic hub devices such as puncture membranes inhibit the introduction of microbes into the catheter lumen. However, this does not protect the external surface of the catheter or around the site of implantation.

Second, an ionic silver composition deposited on the outer surface of the catheter exhibits broad-spectrum antimicrobial activity at the insertion-subcutaneous junction. However, this coating has only minimal release of silver from the surface and does not protect the internal surfaces.

Third, an anticoagulant/antimicrobial lock (flush) that includes a combination of anticoagulant and antimicrobial agents is particularly useful for long-term catheters where hub contamination leads to lumen colonization and, ultimately, to bloodstream infection (18-20). A wide spectrum of antibiotics can be used, but general use of antibiotics in lock solution is limited due to the certainty of inducing bacterial antibiotic resistance. Microbial biofilm is able to inhibit the activity of some glycopeptide antibiotics such as vancomycin, making antibiotic lock solutions less effective (15). Furthermore, this approach does not protect the external surface of the catheter.

Fourth, impregnation of the catheter with an antimicrobial agent has a great advantage because it could protect the external and internal surfaces of the catheter. Since the first reports in the 1980's of oxacillin bonded to polytertrafluoroethylene grafts (21, 22), many other drugs, including antibiotics or chemical molecules, were successfully attached to catheter surfaces.

There are still not enough clinical studies and data to choose the proper strategy for preventing catheter infection because most drugs or antibiotics suitable for use in catheters do not exhibit broad spectrum antibiotic, antiviral, and/or antifungal activity. Several randomized studies of antibacterial-impregnated catheters have failed to show an advantage in preventing catheter infection. On the other hand, prolonged exposure to the immobilized antibacterial agents (as they are released) may lead to the development of bacterial resistance that may be difficult to detect (23). Therefore, the need still exists for new therapeutic agents bonded externally and internally to medical devices such as catheters to destroy the broad range of bacteria strains within biofilm and to prevent infection and/or development of bacterial resistance.

Chronically implanted medical devices are an example of devices in which a long-term antibacterial property would be of great benefit to patients. There are some medical devices which are not implanted but which could also provide more benefit if they had ability to prevent bacterial growth on their surface or within the biofilm building on the surface. An example is dialysis machines used for chronic dialysis therapy. Within dialysis centers these machines are used for years in treatment of many different patients. The dialysate fluid they deliver is rich in nutrients for bacteria, so bacterial growth within the dialysate fluid is a continuing problem. Bacterial growth in the dialysate exceeding 2000 organisms/ml can result in transfer of endotoxin to patients with resulting fever, low blood pressure, nausea, and other symptoms. Bacterial content of the dialysate is measured frequently, but only on randomly selected machines. To diminish bacterial content of the dialysate, each machine is disinfected each night after treatment of several patients. Biofilm builds on all of the hydraulic pathways of the dialysis machine, making disinfection somewhat difficult especially after a heavy bacterial contamination. Most of these pathways are constructed of polymers, either flexible or rigid. If these polymers incorporated organic dyes then bacteria within the surfaces of the hydraulic pathways and the adherent biofilm would be killed continually. The bacterial load of dialysate would therefore be reduced, for all patient treatments.

Looking one step further back, the water system providing pure water for dialysis is a significant source of the bacteria resulting in excessive concentration in dialysate. If the purified water source contains more than 200 bacteria/ml then the machines become contaminated and the dialysate develops high concentrations of bacteria with adverse events described above. The water system contains a number of components to purify water and some to eliminate bacteria. However one component of the system, activated charcoal, removes chlorine from the water, making the rest of the system vulnerable to bacterial growth on polymer surfaces or adherent biofilm. The entire water treatment system contains membranes and pipes made of polymer materials. If these materials were impregnated with organic dyes, then the bacterial content of the pure water would be diminished (and meet established standards) and this would diminish bacterial exposure of dialysis patients.

Shanbrom described a "Disinfectant plastic sponge material" in US patent 5,811,471. However his process was merely to soak a PVA sponge material in an organic dye solution to bind some of the dye to the material.. The effective materials were readily colored by the disinfectant dyes. Furthermore, extensive water washing will be unable to remove the tightly bound dye.. In follow-up patent "Microbicide Treated Polymeric Materials" US 6,183,764 he describes that plastics were lightly colored by organic dye exposure. However again "extensive washing will be unable to remove the tightly adsorbed microbicide". In follow up patent "Microbicide Treated Polymeric Materials" US 6,361,786 he describes the same process, but adds an alternative process for coating the material with organic dye, by pre-treatment of the polymer material with meglumine (an ionic compound) for 24 hours. This process apparently "enhanced binding of organic dyes". The process was suggested to have value in treatment of catheters.

US-A-5 709 672 discloses a medical device, eg a catheter, comprising a silicone or polyurethane polymeric material impregnated with on antimicrobially and antifungally effective amount of gentian violet, the device being prepared by contacting the polymeric material with a gentian violet solution for a predetermined period of time.

### SUMMARY OF THE INVENTION

The present invention relates to a method of manufacturing polymeric material for implantable medical devices that exhibit antimicrobial activity. Various aspects of the invention provide various advantages. While the actual nature of the invention covered herein can only be determined with reference to the claims appended hereto, certain forms and features, which are characteristic of the preferred embodiments disclosed herein, are described briefly as follows.

The present invention provides a method of manufacturing polymeric material for a medical device. The method comprises contacting the polymeric material with a liquid composition comprising a organic dye and a reducing agent for a time sufficient to impregnate the polymeric material with the organic dye and thereafter removing the impregnated polymer from the liquid composition.

Medical devices with the ability to kill bacteria contacting the surface of the device and in the surrounding tissues for many months are provided by placing organic dye compounds within the plastic polymers used in construction of the devices. The medical devices may be implanted or used external to the patient in delivery of fluid to the patient. A method is described for activating organic dyes so that they will avidly absorb into a variety of polymers in a few hours, penetrating through the entire depth of the polymer material used in construction of the medical devices. This process can be performed after extrusion or casting of the polymer material. Because of the large store of organic dye within the polymer, the polymer is bactericidal to any bacteria contacting the material surface but also releases organic dye into surrounding biofilm and tissues, killing bacteria in the vicinity of the surface of the medical device.

In one form the present invention provides a medical device for implantation into tissue of a patient or use in fluid preparation to be delivered to a patient. The device comprises a polymeric material impregnated with an organic dye exhibiting antibacterial activity and which is effective to release the organic dye into contacting media for at least two weeks.

Further objects, features, aspects, forms, advantages and benefits shall become apparent from the description and drawings contained herein.

### BRIEF DESCRIPTION OF THE DRAWINGS

Fig. 1 is a perspective view of one embodiment of a catheter provided in accordance with the present invention.
Fig. 2 is a perspective view of one embodiment of a suture treated in accordance with the present invention.
Fig. 3 is a one embodiment of a surgical staple treated in accordance with the present invention.

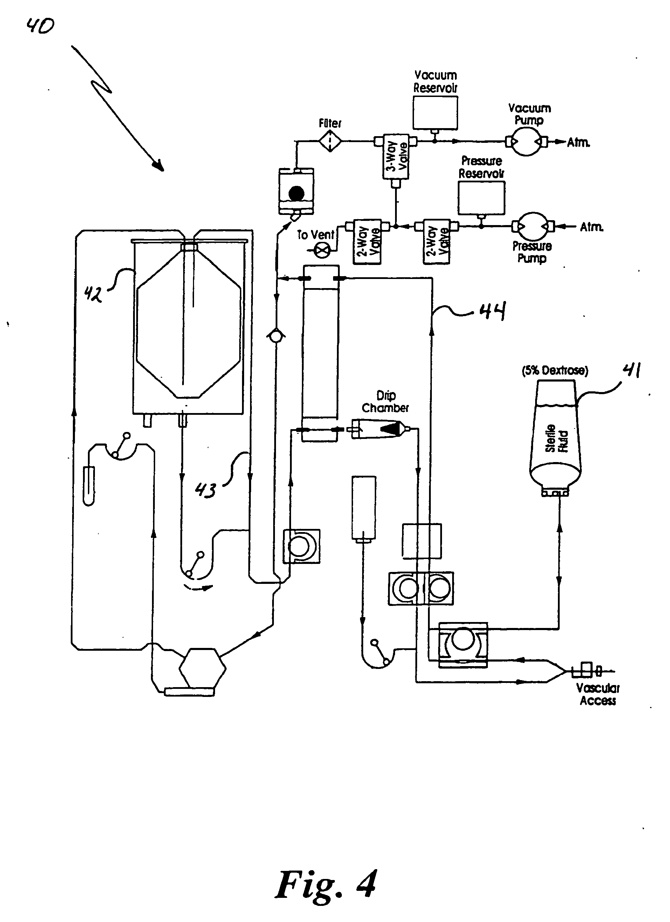

Fig. 4 is a schematic illustration of one embodiment of a dialysis system that can include a variety of treated components in accordance with the present invention.
Fig. 5 is a schematic illustration of the physical design of the cell culture on a growth medium all in contact with a polymeric material treated in accordance with the present invention.
Fig. 6 is a scanned image of a polymeric material treated in accordance with the present invention in contact with a cell culture in a petri dish.
Fig. 7 is a graph illustrating the bactericidal properties of treated polyurethane (EG) and polyurethane/polycarbonate materials (PC) impregnated with methylene blue against E. coli in accordance with the present invention. Control includes a non-impregnated surface (NIS) with contact to the bacteria solution
Fig. 8 is a graph illustrating the bactericidal properties of a polyurethane (EG) and a polyurethane/polycarbonate (PC) material impregnated with methylene blue against S. aureus in accordance with the present invention. Control includes a non-impregnated surface (NIS) with contact to the bacteria solution
Fig. 9 is a graph illustrating the bactericidal properties of a polyurethane (EG) and a polyurethane/polycarbonate (PC) material treated with methylene blue against S. epidermidis in accordance with the present invention. Control includes a non-impregnated surface (NIS) with contact to the bacteria solution
Fig. 10 is a scanned image of a petri dishes after culture of bacterial solution contacting control surfaces, demonstrating bacteria having a colony count of 10⁷ to 10⁸.
Fig. 11 is a scanned image of a petri dish having a near zero colony count of bacteria, which was obtained after incubation of bacterial solutions with a polyurethane/polycarbonate material impregnated with methylene blue in accordance with the present invention.
Fig. 12 is a scanned image of several polymeric tubes that have been impregnated with a methylene blue in combination with an activating agent in accordance with the present invention.
Fig. 13 is a scanned image of several polymeric tubes that have been immersed in a solution of methylene blue after pretreatment of the tubes with N-methylglucamine and without any activating agent.

### DETAILED DESCRIPTION OF THE INVENTION

For the purposes of promoting an understanding of the principles of the invention, reference will now be made to the embodiments illustrated herein and specific language will be used to describe the same. It will nevertheless be understood that no limitation of the scope of the invention is thereby intended. Any alterations and further modifications in the described processes, systems, or devices, and any further applications of the principles of the invention as described herein, are contemplated as would normally occur to one skilled in the art to which the invention relates.

In general, medical devices produced from polymeric material manufactured by the method according to the present invention exhibit antimicrobial and/or antiviral properties. The medical devices include a portion formed of a polymeric material or matrix that has been impregnated with an organic dye compound. The organic dye can be selected from: methylene blue, toluidine blue, methylene violet, azure A, azure B, azure C, brilliant cresol blue, thionin, methylene green, bromcresol green, gentian violet, acridine orange, brilliant green, acridine yellow, quinacrine, trypan blue, and trypan red. Many of these dyes have a phenothiazine ring structure, acridine ring, or similar structure. Most of these dyes have the ability to be an electron donor or electron receptor in oxidation-reduction reactions, and also have a change in oxidative potential on exposure to light (being photo-oxidants).. In preferred embodiments, the organic dye compound is bound to carbon chains or embedded or absorbed into the polymeric matrix as an inclusion complex. The polymeric material can slowly release the absorbed organic dye compound into the surrounding tissue or fluid. The rate of release can extend over one, two, or more months. The rate of release can be controlled by controlling the amount of organic dye compound absorbed into the polymeric material.

It has also been determined that the treated devices do not lose efficacy upon extended storage. For example, the devices can be prepared and stored either in a sterile container or in clean packaging until needed. When desired, the devices can be sterilized, if necessary, and then immediately used or implanted in patients. Upon implantation or contact with surrounding tissue, the treated polymeric begins to immediately release the absorbed organic dye complex at a substantially steady rate as evidenced by the resulting antimicrobial activity of the devices. Examples of implantable medical devices that can be used and treated in accordance with the present invention include: catheters (either temporary or long-term indwelling medical catheters), sutures, surgical staples, intrauterine devices, vascular stents, catheter connectors, connector caps, subcutaneous or transcutaneous ports, contact lenses, implanted artificial lenses, pacemaker leads, pacemakers, artificial hearts, implantable lungs, implantable infusion pumps and numerous other implantable medical devices. Examples of external medical devices with advantage from this process include dialysis machines, dialysis water delivery systems, water circuits within the dialysis unit, water delivery systems for respirator therapy, and water or fluid delivery systems for any other medical use if used for extended periods of time..

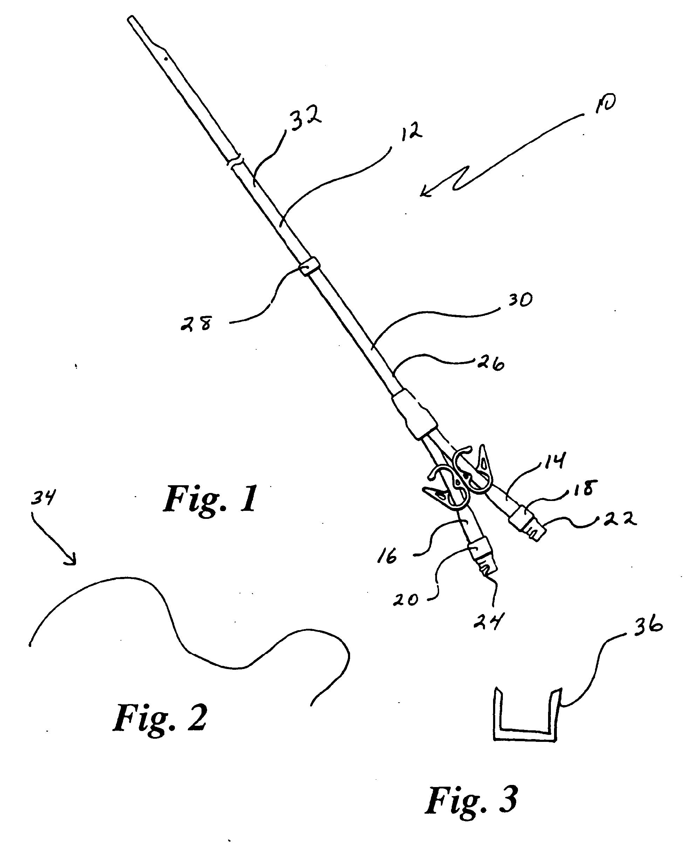

Fig. 1 is a perspective view of one embodiment of a medical device 10 including a catheter 12. Catheter 12 includes a first and second lumens 14 and 16, respectively. Each lumen 14 and 16 includes a hub 18 and 20 and a puncture cap 22 and 24. The lumens 14 and 16, the hubs 18 and 20, and the puncture caps 22 and 24 can be formed of the same or different polymeric materials. An outer sheath 26 surrounds a portion of lumens 14 and 16. A cuff 28 encircles sheath 26. Additionally, the lumens 14 and 16 and hubs 18 and 20 can be attached using a biocompatible glue (not shown). The treatment of medical devices such as catheter 12 according to the present invention provides distinct advantageous. For example, each of the components of catheter 12 can be treated and impregnated with the organic dye compound regardless of the polymeric material used to form the components. Consequently, each component can exhibit antimicrobial activity. Additionally, if desired, selected portions of catheter 12 need not be treated. For example, lower portion 32 of sheath 26 can be left untreated, while cuff 28 and the implantable, upper portion 32 can be treated with the organic dye compound.

After impregnation with the organic dye compound, catheter 12 has a distinctly dark color. If one or more of the lumens 14 and 16 and/or sheath 26 were cut through, it would be readily apparent that the polymeric material of these components has been completely impregnated with the organic dye. The depth of impregnation can be controlled by the concentration of dye, the length of treatment, and/or the concentration of the activating agent and, optionally, the temperature at which the polymeric material is treated. In preferred embodiments, the polymeric material is completely impregnated with the organic dye and exhibits a dark color from the outer surface through to the inner surface.

Figs. 2 and 3 illustrate other implantable medical devices that be treated and prepared in accordance with the present invention. Fig. 2 is s perspective view of surgical suture material 34 that impregnated with a dye. Figure 3 is an illustration of a surgical suture 26.

Fig. 3 is a schematic illustration of a dialysis machine 40 that contains various components that can be treated in accordance with the present invention. Dialysis unit 40 includes various flexible and non-flexible polymeric components that can contain a variety of fluids. For example, container 41 and 42. Additionally various plastic tubing such as tubing 43 and 44, which are hydraulic pathways within the unit, can be treated with a dye in accordance with the present invention.

The treated material is prepared to release organic dye at a very slow rate over months or years of use. Experiments have indicated that if a catheter is treated by the process according to the present invention to impregnated the organic dye, such as methylene blue, throughout the entire body of the polymeric material, then placing the catheter within a 1 liter volume of normal saline resulted in leaching of the dye over a one month period, turning the entire volume to an intense blue color. When the saline is replaced with fresh saline, exactly the same intense blue color developed in the fresh saline solution during the second month. This process of replacing the saline solution can be continued, that is, replacing the saline solution with fresh solution monthly, for at least nine months. The color resulting during the sixth month in the fresh saline solution is a light blue. If the process is continued for an additional 3 months or up to a total of 9 months the resulting fresh saline solution is light blue in color, but the release of the dye continues. This indicates that the treated catheter is effective to release the antimicrobial dye at a rate and a concentration sufficient to inhibit microbial and/or bacteria growth for at least up to 9 months.

The medical devices prepared according to the present invention can be prepared by contacting the selected polymeric portion with a solution containing the organic dye compound or dye and a reducing agent. The solvent for the solution can be water or saline. Alternatively, other solutions can be used including, but not restricted to: alcohol, for example, ethanol or isopropyl alcohol; polar organic solvents, for example, chloroform; methylene chloride; acetone, tetrahydrofuran (THF); and mixtures of these solvents or other solvents as could be readily determined by those skilled in the art. The solvent can be selected based upon the nature of the polymeric material. It is particularly important to select a solvent that will not degrade or partly dissolve the polymeric material or any glue adhering the material to the medical device.

The organic dye compound and the reducing agent are added or suspended in the solvent. The order of addition is not critical. The organic dye compound and activator are present in amounts sufficient to impregnate the polymer within a desired amount of time. Preferably the organic dye compound is provided in an amount ranging between about 0.1 and 1.0 weight percent (wt%). More preferably, the organic dye is provided in an amount between about 0.1 and about 0.3 wt%. The reducing agent can be provided in amounts ranging between about 0.01 and 3 wt%; more preferably, between about 1.0 and about 1.0 wt%. A buffer can also be included in the solution to maintain a pH of between about 4 and about 9. The buffer can be a commonly available buffering compound, for example, a citrate buffer, and can be readily selected by one skilled in the art. The temperature of the solution can be maintained between about 20 °C and slightly above ambient (25 C) temperature. Higher temperatures can be utilized; however, this may significantly degrade and/or deform the polymeric material.

The polymeric material is immersed in the solution described above. The material can be maintained in the solution for a time sufficient to substantially impregnate the polymeric material and provide a substantially homogenous distribution of the organic dye throughout the polymer. The time can vary depending upon the concentration of organic dye compound, the reducing agent, and the polymer thickness. In preferred embodiments, the polymeric material can be immersed in the solution from a time ranging between about one minute to several hours.

When desired, the polymeric material is removed from the solution. It has been observed that upon initially removing the polymeric material from the solutions of low organic dye concentration, there is no immediate, noticeable color change on the surface of the polymer. This material is then washed repeatedly with the solvent and/or a neutral physiological saline solution to remove any residual, non-bound organic dye compound or reducing agent. The material is washed until the wash water exhibits no discoloration due to the organic dye or compound. Within a few minutes after washing and drying the polymeric material, the surface of the material begins to significantly darken. The treated material can then be stored until needed. Polymers exposed to higher concentrations of organic dye emerge from the treatment already colored by the dye, though the color may increase over time and/or exposure to air.

It has been determined that medical devices prepared according to the present invention can be stored for several months without any loss of efficacy; the stored devices maintain the antimicrobial properties.

In preferred embodiments, the organic dye for use in the present invention can be selected among: methylene blue, toluidine blue, methylene violet, azure A, azure B, azure C, brilliant cresol blue, thionin, methylene green, bromcresol green, gentian violet, acridine orange, brilliant green, acridine yellow, quinacrine, trypan blue, trypan red and mixtures of these compounds.

Specific examples of reducing agents for use in the present invention include ascorbic acid, ferrous ions, and other reducing agents. Examples of ferrous ions include ferrous salts, such as ferrous gluconate.

Polymeric materials that can be used with the present invention can be biodegradable (or resorbable polymers) and non-biodegradable polymers. Examples of non-biodegradable polymers that can be used in the present invention include, but are not restricted to: acrylics, polyacrylates, polymethacrylates, fluorocarbons, hydrogels, polyacetals, polyamides (nylons), polyurethane/polycarbonates, polyesters, poly(ether, ketones) (PEK), polyimides, polyolefins, polystyrene, polysulfones, polyurethanes, polyvinyl chloride (PVC), silicone rubbers, polyethylene, polyurethane, latex, polyesters, poly(ethylene-terephthalate), and blends of these polymers. Examples of biodegradable polymers for use in the present invention include, but are not restricted to: poly(amino acids), polyanhydrides, polycaprolactones, poly(lacti-glycolic acid), polyhydroxybutyrates, polyorthoesters, and blends of these polymers. The polymers for use in the present invention can be polymer blends, homopolymers, and/or copolymers. Use of the term co-polymers is intended to include within the scope of the invention polymers formed of two or more unique monomeric repeating units. Such co-polymers can include random copolymers; graft copolymers; block copolymers; radial block, diblock, and triblock copolymers; alternating co-polymers; and periodic co-polymers. Use of the term polymer blend is intended to include polymer alloys, semi-interpenetrating polymer networks (SIPN), and interpenetrating polymer networks (IPN).

In use, the medical devices prepared according to the present invention are preferably sterilized immediately before implantation into the patient. Upon implantation according to standard surgical procedures, it can be observed that there may be a slight discoloration around the site of implantation. Additionally, contacting the polymeric material with normal saline or other solvents may induce an added release of the organic dye from the polymeric material. For example, in catheter 12 illustrated in Fig. 1, wiping the hubs 18 and 20 and puncture caps 22 and 24 with BETADINE® and/or an alcohol pad may cause discoloration of the respective pads. Additionally the organic dye compound will leach into any lock solution in the catheter lumen. However, the catheter as illustrated in Fig. 1 can be used according to standard medical practices. Furthermore, lock solutions for these catheters can include normal saline, antimicrobial/antibiotic compositions, and anticoagulents, such as heparin or a citrate composition as has been used in the past.

The medical devices prepared according to the present invention can also be prepared by combining one or more of the organic dyes and a reducing agent with a polymeric material prior to manufacturing the medical device. For example, the solid organic dye and activating agent can be combined and mixed with a pellitized polymer to provide an extrudable mixture, which is subsequently extruded or molded into the desired implantable medical device.

As noted above, the organic dye can be selected among: methylene blue, toluidine blue, methylene violet, azure A, azure B, azure C, brilliant cresol blue, thionin, methylene green, bromcresol green, gentian violet, acridine orange, brilliant green, acridine yellow, quinacrine, trypan blue and trypan red, or combinations of these compounds. Preferred dyes include dyes of the phenothiazine class. Methylene blue is a water-soluble phenothiazine dye used orally as a urinary tract antiseptic or parenterally as an antidote for cyanide poisoning or drug-induced methemoglobinemia. It also has been used for more than 50 years as an intravenous injection to define the course of ureters during pelvic surgery. Methylene blue collectively with other dyes from this family, such as toluidine blue and methylene violet, are effective inactivators of pathogenic organisms including viruses, bacteria, and yeast in skin lesions, especially when photo-activated on skin lesions (24). In the last decade, methylene blue has been used for inactivation of a variety of viruses in fresh frozen plasma (25-27), whereas toluidine blue has been reported as an antifungal and antibacterial drug for inactivation of yeast and some bacteria (28-31). In 2001 a comparison study of methylene blue and toluidine blue bactericidal efficacy after photoactivation against gram-positive and gram-negative microorganisms was published (32). The report pointed out many factors involved in destruction of bacteria such as concentration, partition coefficient, photodynamic efficacy, light intensity and exposure time or dimers formation. However, both organic dye also revealed sufficient bactericidal potency when experiments were performed in the dark. These dyes are to some extent amphipathic and cationic. Consequently, they contain a hydrophobic portion that can interact preferentially with lipids or other hydrophobic substances and a positively charged portion that interacts with water or negatively charged surfaces. Driven by electrostatic attraction to the negatively charged cell membranes of microbial targets, these dyes enter the membranes, form new channels and pores, and change the permeability that eventually can kill the microbes. It is thought that these dyes can interfere with the vital intracellular reactions involving oxidation and reduction such as conversion of NADH to NAD and vice-versa.

Methylene blue has an advantage as a catheter-coating material because of its extensive use as an IV and topical medication. This means that safety issues are less likely to arise than with many other dyes.

In the impregnation process, two reducing agents were used, ascorbic acid and ferrous gluconate, in a similar range of concentration. Ascorbic acid appeared to react faster and provide more reproducible results. Many intermediate species are created during the oxidation-reduction process by methylene blue after activation by ascorbic acid, and these intermediates can help to impregnate the matrix of the plastic materials. From the clinical results, it appeared that activation of methylene blue by either ascorbic acid or ferrous gluconate afforded nearly identical antibacterial properties to the treated plastic material.

It is also believed that application of methylene blue or toluidine blue as a long-term coating compound for CVC catheters does not require light activation to be effective. The treated material exhibits sufficient bactericidal activity in the dark.

Light activation of the treated material may be advantageous for imparting antiviral and enhanced antibacterial activity. The rate of bactericidal activity can be enhanced by room light, which penetrates the external tubing of the catheter. Shining a very bright light down the lumen of a CVC catheter may also further increase bactericidal action. It is thought that methylene blue can transfer energy that it picks up from the light to molecular oxygen, so oxygen in the blood might have an effect similar to light. The singlet oxygen, which is formed, can mediate nucleic acid damage, principally at guanosine sites in the DNA or RNA backbone, and cause genetic sterilization. Light was not completely excluded from the above-described experiments; however, incubation was performed in the dark, plating of bacteria in Petri dishes was performed in low-level room light, and the cultures were maintained in complete darkness. The molecular structure of these dyes, positive charge and possible dimerization, may allow methylene blue and similar dyes to kill bacteria by changing the transmembrane permeability of microbes. This function does not require activation by light. If the concentration of methylene blue increases inside the bacteria cell, other factors result in damage of bacteria, including changes in the redox potential due to excited states of the dye, quantum yield of the triplet-state formation, and quantum yield of singlet oxygen formation.

For the purpose of promoting further understanding and appreciation of the present invention and its advantages, the following Examples are provided. It will be understood, however, that these Examples are illustrative and not limiting in any fashion.

### Example 1: Impregnation of Polymeric Materials

Two different plastic materials that are currently used for CVC catheter production were impregnated with methylene blue. The first polymer was EG-85A from the Tecoflex family of aliphatic polyurethanes (EG) and the second was PC-3575A from the Carbothane family of aliphatic polyurethane/polycarbonates (PC). Neither of the plastics tested were observed to become impregnated by methylene blue when exposed for up to 24 hours to 0.1 to 0.5 % of methylene blue solution in the range pH between 4 to 9 units. Fluorescent light did not help to impregnate methylene blue on either plastic. It has been discovered, however, that the rate of plastic impregnation with methylene blue is significantly increased by employing an activating agent (or "activator") such as, for example, a reducing agent. Examples of reducing agents include ascorbic acid or a soluble form of ferrous ion (for example, ferrous gluconate).

Ascorbic acid is a powerful reductant and free radical scavenger. Kinetics of oxidation of ascorbic acid by methylene blue in acid media revealed many steps and several intermediate active species of methylene blue and ascorbic acid (33-35). It is believed that at least some of these reactive intermediates can activate the carbon atoms of the polymeric matrix. This process in turn allows binding of methylene blue to the polymer chains. Experiments performed in 0.24 M citrate buffer at pH 4.5 with 0.1 % methylene blue and 1%-2% of ascorbic acid or ferrous gluconate permitted impregnation of both polymers within a few hours at ambient temperature. It was observed that the organic dye compound penetrated deeper into polyurethane than into polyurethane/polycarbonate; however, the polyurethane/polycarbonate was impregnated sufficiently to penetrate the surface of the plastic material.

The data from the two different materials revealed that the active form of methylene blue, which is bound to a matrix, is probably the leuco-form. When exposed to air after washing the excess of reagents, the bound methylene blue is gradually oxidized to oxy form. The properties of the absorbed layer, such as thickness, dimerization, and hydrophilicity, may be controlled by proper selection of dye and activator concentration used in this composition and time of reaction. The bonding is strong but not so much as to prevent a small amount of leaching in aqueous solutions or saline. This process can be observed to occur over weeks and months and may have great advantages including bactericidal activity in the biofilm and surrounding tissues or clots.

### Example 2: Antimicrobial Evaluation

The polyurethane- and polyurethane/polycarbonate-impregnated plastic materials were tested against three strains of bacteria: E. coli 25922, S. epidermidis 49134, and S. aureus 29213. The impregnated plastic materials were prepared as described above. Inoculum of each bacterium was prepared from a single colony in 15 ml of trypticase-soy (TS) medium overnight at 37°C. Fifteen µl of inoculum was added to 15 ml of fresh medium and incubated for a few hours (5-6 h). Seventy-five (75) µl of 100 times diluted fresh culture was then used in experiments with samples of each of the impregnated plastic material. A culture of the selected strain was placed on the bottom of a petri dish and covered by an approximately 2.25 cm² sheet of the impregnated plastic material. The sheet was pressed firmly against the agar to get a thin and equal layer of culture medium contacting the sheet. A small container with 1 ml of water was placed in the upturned lid of the petri dish. The petri dish with the inoculum and plastic sheet were then inverted and mated with the lid. The lid and petri dish were sealed tightly by coating the rim of either the lid or the dish with petroleum jelly. The dish was then placed upside down in an incubator maintained at 32°C for 24 hours.

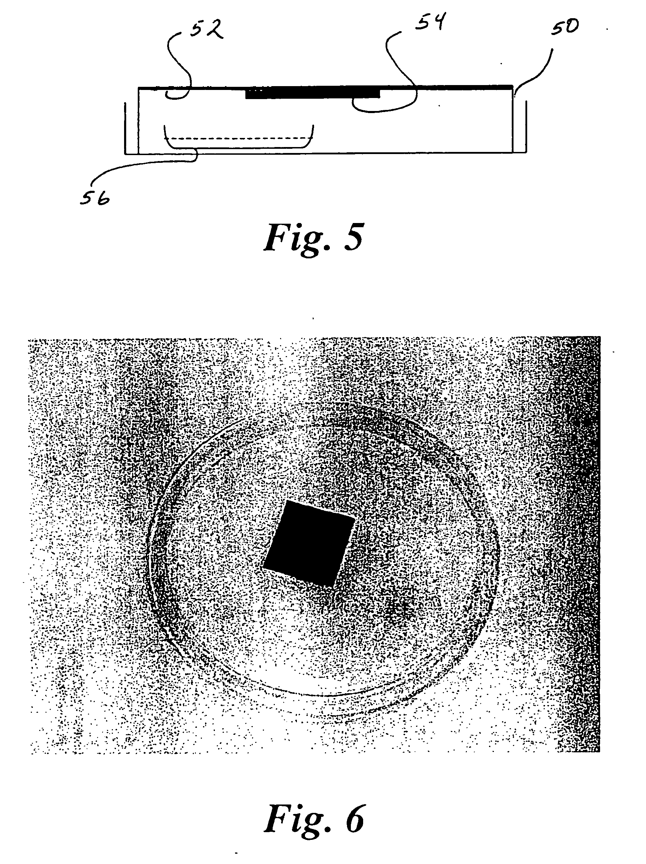

The physical design of the experiments is shown in Figs. 5 and 6. Referring specifically to Fig. 5, the petri dish 50 includes an agar 52 onto which the bacteria was spread. A treated plastic sheet 54 lies on the agar 52. A container 56 with water is placed in the upturned lid 58.

Simultaneously, controls were created for the experiment. Untreated polyurethane and polyurethane/polycarbonate sheets (-2.25 cm²) were placed in separate petri dishes to culture suspension. Another control was 75 µl of bacteria culture placed in the petri dish as a "hanging drop" without any plastic sheet material. As described above, the dishes were mated to their lids, which contained 1 ml of water, sealed, and placed in the incubator.

After approximately 24 hours of incubation at 32° C, 15 µl of bacterial cultures from each dish were mixed with 15 ml of fresh TS. Resulting suspensions were used as "stock solutions". The stock solutions were further diluted 10², 10⁴, 10⁶ fold. One ml of each dilution was spread on a separate TS agar plate incubated overnight at 37°C for colonies calculation.

All three tested strains of bacteria revealed dramatic inhibition of culture growth after contacting methylene blue-impregnated plastics sheets. There appeared to be no significant differences between the polyurethane- or polyurethane/polycarbonate-impregnated materials. The log₁₀ CFU reduction after 24 hours of incubation is a range of 7 to 6. The residual bacterial activity was very low. Bacteria colonies were detected in only two of the 1 ml of stock solutions when incubated on the agar plates.

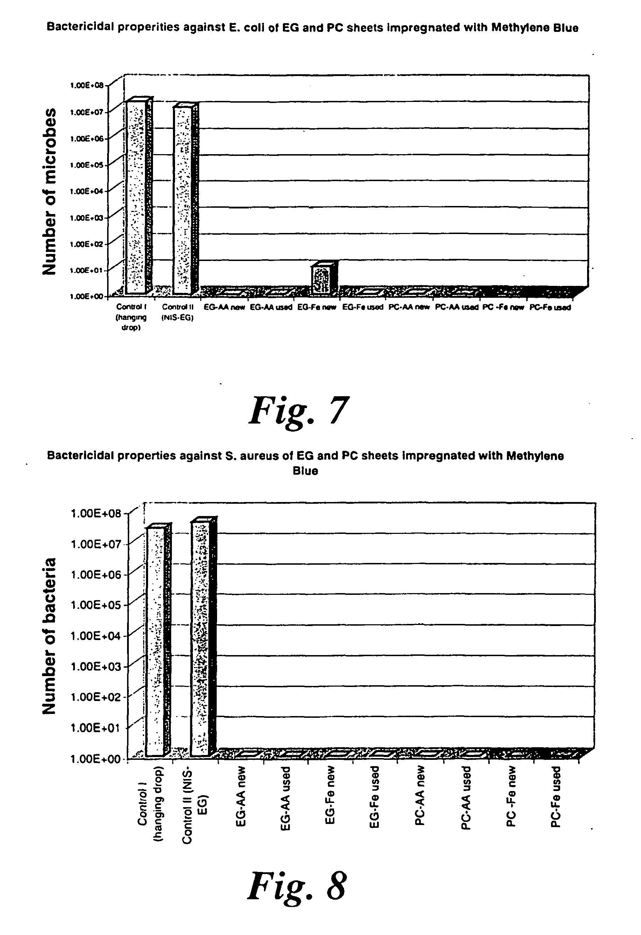

Fig. 7 is a graph illustrating E. coli bacterial concentration in the control samples and in the media in contact with the impregnated polyurethane sheets. The concentration of E. coli bacteria in each of the controls was 10⁷ to 10⁸ bacteria per ml. The bacterial concentrations was nearly zero or non-detectable on the media in contact with the four methylene blue-impregnated membranes: EG-AA (polyurethane with ascorbic acid as the reducing agent), EG-Fe (polyurethane with ferrous ion as the reducing agent), PC-AA (polyurethane/polycarbonate with ascorbic acid as the reducing agent), and PC-Fe (polyurethane/polycarbonate with ferrous ion as the reducing agent). In separate experiments, samples of each type of membrane were stored dry for one month and re-tested as above described. For all of the impregnated sheets, whether activated by ascorbic acid or ferrous ion, the results of the later tests were the same as if the impregnated plastics were freshly prepared. Each treated sheet allowed nearly zero bacteria growth in the contacting media.

Fig. 8 is a graph illustrating the results of experiments as described above except using S. aureus as the bacteria strain. Results are substantially the same as found for E. coli above. For all of the impregnated sheets, whether activated by ascorbic acid or ferrous ion, the results were the same: nearly zero bacteria in the contacting fluid. As before in separate experiments, samples of the impregnated sheets were stored dry for one month and retested. Again, the test results were essentially identical with those previously obtained for the freshly prepared impregnated sheets.

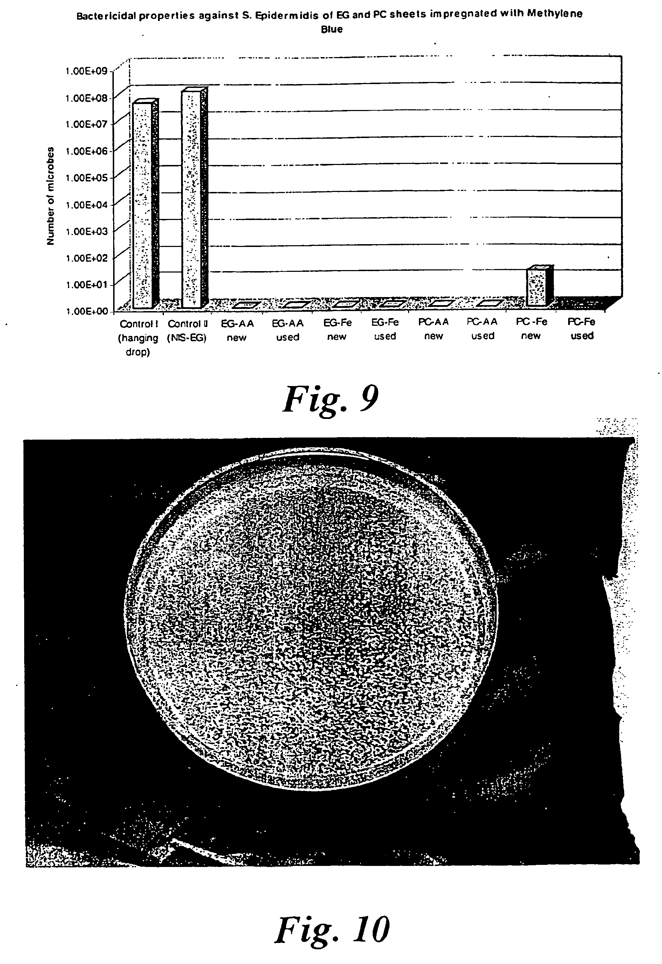

Fig. 9 is a graph illustrating the results of experiments as described above using S. epidermidis as the bacteria strain. As can be seen from the graph, the results are substantially the same as those obtained for the E. coli and S. aureus strains. For all of the impregnated plastic sheets, whether activated by ascorbic acid or ferrous ion, the results were the same: nearly zero bacteria growth in the contacting media. As before, samples of the impregnated sheets were stored for one month and retested. The results again were essentially identical to those obtained with the freshly prepared impregnated sheets.

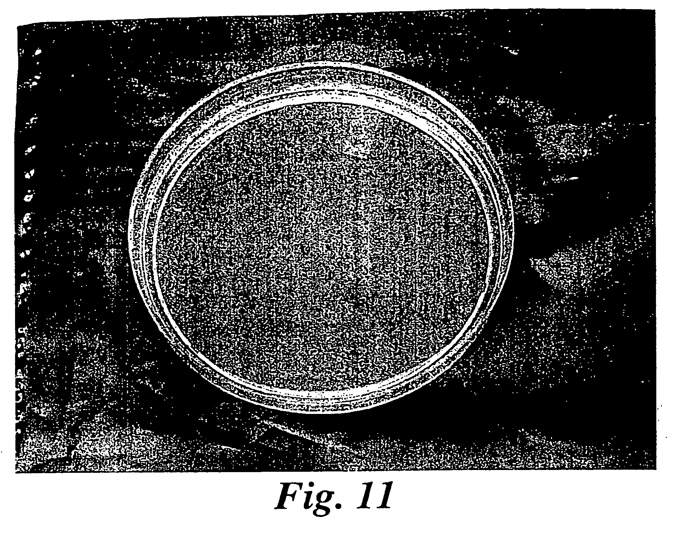

Figs. 10 and 11 illustrate the dramatic visual difference of a log₁₀ reduction of 6-7 in bacteria growth. Fig. 10 is a scanned image of a petri dish with a colony count of 10⁷ to 10⁸. This is one of the control plates from the above set of experiments after appropriate dilution. Fig. 11 is a scanned image of a petri dish after culturing media using the same dilution factor of bacteria cultures exposed to the methylene blue-impregnated plastics. The colony count is essentially zero with only scattered colonies observable on the agar plate.

### Example 3: Comparison of Polymers Treated with a Dye and with a Dye In combination with an Activating Agent

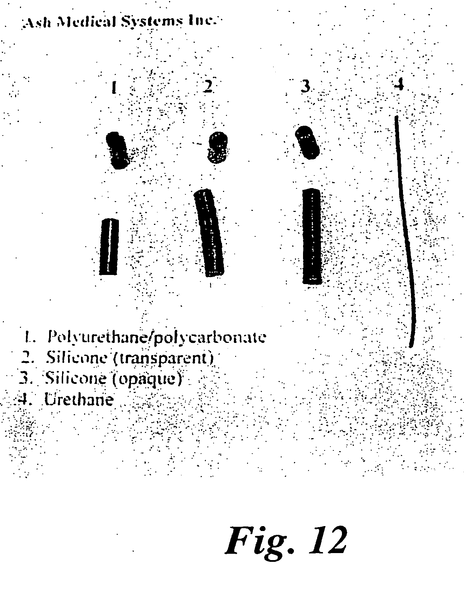

A series polymeric tubing were treated according the present invention as described above in Example 1, as follows: the tubings were immersed in a 0.1% aqueous solution of methylene blue and 2% solution of ascorbic acid in 0.24 M citrate buffer (ph 6) for two hours, then removed, washed with saline and dried. Figure 12 is a scanned image of the resulting treated polymeric tubing

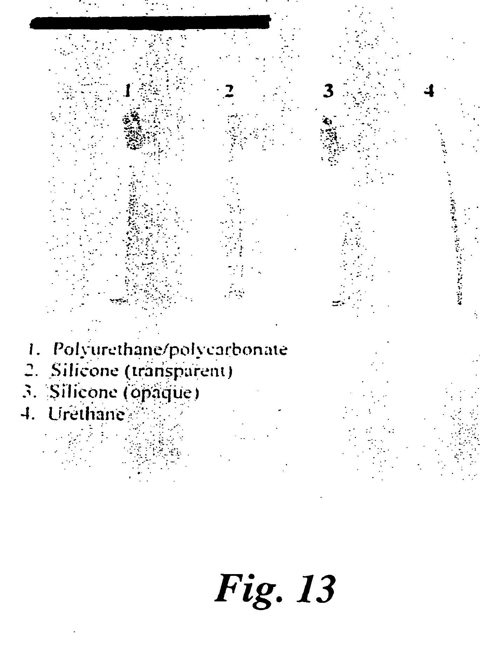

For comparison, the same type of polymeric tubing was treated using different methods of treatment. A set of the tubing was treated: the tubings were immersed in a 1% aqueous solution of methylene blue for 24 hours, then removed, washed with saline and dried. Appearance was similar to tubings shown in Fig 13.

Another set of the tubing was treated with a mixture of methylene blue and N-methylglucamine as follows: the tubings were immersed in a 1 % aqueous solution of N-methylglucamine for 24 hours, washed with saline and dried and immersed in 1% aqueous solution of methylene blue for 24 hours, then removed, washed with saline and dried. Figure 13 is a scanned image of the resulting treated polymeric tubing.

The results of these experiments are listed in Table 1 below.

**Table 1**

| Material | Methods | | |
|---|---|---|---|
| | 1 % Methylene Blue (24 hr) | 1% N methylglucamine (24 h), then 1% Methylene Blue (24 h) | 2% Ascorbic Acid and 0.1 % Methylene Blue (2 hr) |
| Polyurethane | | Very light blue on he surface only | Strong dark blue throughout material |
| Polyurethane/ polycarbonate | Slightly dirty white on the surface | Very little of white on the surface | Strong dark blue throughout material |
| Silicone (transparent) | Very light green on the surface | Very light green on the surface | Strong dark blue throughout material |
| Silicone (opaque) | Tint of green on the white surface | Tint of green on the white surface | Dark blue throughout material |

The present invention contemplates modifications as would occur to those skilled in the art. It is also contemplated that processes embodied in the present invention can be altered or added to other processes as would occur to those skilled in the art without departing from the scope of the present invention.

Further, any theory of operation, proof, or finding stated herein is meant to further enhance understanding of the present invention and is not intended to make the scope of the present invention dependent upon such theory, proof, or finding.

While the invention has been illustrated and described in detail in the drawings and foregoing description, the same is considered to be illustrative and not restrictive in character, it is understood that only the preferred embodiments have been shown and described and that all changes and modifications that come within the scope of the invention are desired to be protected.

### References

1. Maki DG. Infections caused by intravascular devices used for infusion therapy: pathogenesis, prevention, and management. In: Bisto AI, Waldvogel FA, eds. Infections associated with indwelling medical devices, 2nd edn. Washington, DC: American Society for Microbiology, 1994; 155-205.
2. Raad II, Bodey GP. Infectious complications of indwelling vascular catheters. Clin Infect Dis. 1992; 15: 197-210.
3. Maki DG. Infections due to infusion therapy. In:Bennett JV, Brachman PS, eds. Hospital Infections. Boston: Little Brown; 1992; 849-898.
4. Jarvis WR, Edwards JR, Culver DH, et al. Nosocomial infection rates in adult and pediatric intensive care units in the United States. Am J Med. 1991;9(suppl 3B): 185S-191S.
5. Collignon PJ. Intravascular catheter associated sepsis: a common problem. The Australian Study on Intravascular Catheter Associated Sepsis. Med J Aust. 1994; 161: 374-378.
6. Maki DG, Cobb L, Garman JK, et al. An attachable silver impregnated cuff for prevention of infection with central venous catheters: a perspective randomized multicenter trial. Am J Med. 1988; 85: 307-314.
7. Heiselman D. Nosocomial bloodstream infections in the critically ill. JAMA 1994; 272: 1819-1820.
8. Arnow PM, Quimosing EM, Beach M. Consequences of intravascular catheter sepsis. Clin Infect Dis. 1993; 16: 778-784.
9. Mermel LA, McCormick RD, Springman SR, Maki DG. The phatogenesis and epidemology of catheter-related infection with pulmanory artery Swan-Ganz catheters: a prospective study utilizing molecular subtyping. Am J Med. 1991; 91: 1975-2055.
10. Maki DG, Stolz S, Wheeler S, Mermel LA. A prospective, randomized trial of gauze and two polyurethane dressing for site care of pulmanory artery catheters: implication for catheter management. Crit Care Med. 1994; 22: 1729-1737.
11. Maki DG, Stolz S, Wheeler S, Mermel LA. Prevention of central venous catheter-related bloodstream infection by use an antiseptic-impregnated catheter. A randomized, controlled trial. Ann Intern Med. 1997; 127: 257-266.
12. Costerton JW, Irvin RT, Cheng KR. The bacterial glycocalyx in nature and disease. Annu Rev Microbial. 1981; 35: 299-324.
13. Pfaller MA, Messer SA, Hollis RJ. Variation in DNA subtype, antifungal susceptibility and slime production among clinical isolates of Candida parapsilosis. Diagn Microbiol Infect Dis. 1995; 21: 9-14.
14. Sheth NK, Franson TR, Sohnle PG. Influence of bacterial adherence to intravascular catheters on in vitro antibiotic susceptibility. Lancet. 1985; 2: 1266-1268.
15. Farber BF, Kaplan MH, Clogstron AG. Staphylococcus epidermidis extracted slime inhibits the antimicrobial action of glycopeptide antibiotics. J Infect Dis. 1990; 161: 37-40.
16. Raad II, Costerton JW, Sabharwal U, et al. Ultrastructural analysis of indwelling vascular catheters: a quantitative relationship between luminal colonization and duration of placement. J Infect Dis. 1993; 168: 400-407.
17. Raad II, Luna M, Sayed-Ahmed MK, et al. The relationship between the thrombotic and infectious complications of central venous catheters. JAMA 1994; 271: 1014-1016.
18. Linares J, Sitges-Serra A, Garau J, et al. Phatogenesis of catheter sepsis: a prospective study with quantitative and semiquantitative cultures of catheter hub and segment. J. Clin Microbiol. 1983; 21: 357-360.
19. Salzman MB, Isenberg HD, Shapiro JF, et al. A prospective study of the catheter hub as the portal of entry for microorganisms causing catheter-related sepsis in neonants. J Infect Dis. 1993; 167: 487-490.
20. Stiges-Serra A, Puig P, Linares J, et al. Hub colonization as the initial step in an outbreak of catheter-related sepsis due to coagulase negative staphylococci during parenteral nutrition JPEN 1984; 8: 668-672.
21. Greco RS, Harvey RA. The role of antibiotics bonding in the prevention of vascular prosthetic infections. Ann Surg. 1982; 195: 168-171.
22. Harvey RA, Greco RS. The noncovalent bonding of antibiotics to a polytetrafluoroethylene benzalkonium graft. Ann Surg. 1981; 194: 642-647.
23. Sierdazki K, Roberts RB, Haber Sw, Tomasz A. The development of vancomycin resistance in a patient with methicillin-resistant Staphylococcus aureus infection. N Engl J Med. 1999; 340: 517-523.
24. Wianwright M. Non-porphyrin photosensitizers in biomedicine. Chem Soc Rev. 1997; 25: 351-359.
25. Mohr H, Bachmann B, Klein-Struckmeier A, Lambrecht B. Virus inactivation of blood products by phenothiazine dyes and light. Photochem Photobiol. 1997; 65: 441-445.
26. Lambrecht B, Mohr H, Knuver-Hopf J, Schmitt H. Photoinactivation of viruses in human fresh plasma by phenothiazine dyes in combination with visible light. Von Sang 1991; 207-213.
27. Wagner SJ, Robinette D, Storry J, et al. Differential sensitivity of viruses in red cell suspension to methylene blue photosensitization. Transfusion 1994; 34: 521-526.
28. Ito T. Toluidine blue: The mode of photodynamic action in yeast cells. Photochem Photobiol. 1977; 25: 47-53.
29. Wakayama Y, Takagi M, Yano K. Photosensitized inactivation of E. coli cells in toluidine blue light system. Photochem Photobiol. 1980; 32: 601-605.
30. Wilson M. Bactericidal effect of laser light and its potential use in the treatment of plaque-related diseases. Int Dent J. 1994; 44: 181-189.
31. Bhatti M, MacRobert A, Meghji S, et al. A study of uptake of toluidine blue O by Porphyromonas gingivalis and the mechanism of lethal photosensitization. Photochem Pchotobiol. 1998; 68: 370-376.
32. Usacheva MN, Teichert MC, Biel MA. Comparison of the methylene blue and toluidine blue photobactericidal efficacy against gram-positive and gram-negative microorganisms. Laser Sur Med. 2001; 29: 165-173.
33. Strizhak PE. Kinetics of oxidation of ascorbic acid by methylene blue in acid solutions. Theor Exper Chem. 1993; 29: 283-286.
34. Srtizhak PE. Effect of copper (II) ions on kinetics of ascorbic acid oxidation by methylene blue.
35. Khan MN, Sarwar A. The influence of transition metal ions on the kinetics of ascorbic acid oxidation by methylene blue in strongly acidic media. Turk J Chem. 2001; 25: 433-440.
36. Ash SR, Mankus RA, Sutton JM, Criswell RE, Crull C, Velasquez K, Smeltzer B, Ing T. Concentrated sodium citrate (23%) for catheter lock. Hemodialysis International 4:22-31, 2000.

## Claims

1. A method of manufacturing polymeric material for a medical device, said method comprising:
contacting the polymeric material with a liquid composition comprising an organic dye and a reducing agent for a time sufficient to impregnate the polymeric material with the organic dye ; and
removing the impregnated polymer from the liquid composition.

2. The method of claim 1 wherein said contacting comprises immersing the polymeric material in the liquid composition for a time selected to be between about one minute and about 24 hours.

3. The method of claim 2 wherein said contacting comprises immersing the polymeric material in the liquid composition for a time selected to be between about 60 minutes and about 240 minutes.

4. The method of claim 1 wherein the liquid composition is an aqueous composition.

5. The method of claim 1 wherein the liquid composition comprises a solvent selected from the group consisting of: water, an alcohol, tetrahydrofuran, acetone, and mixtures thereof.

6. The method of claim 1 wherein the polymeric material comprises a polymer selected from the group consisting of: acrylics, polyacrylates, polymethacrylates, fluorocarbons, hydrogels, polyacetals, polyamides, polyurethane/polycarbonates, polyesters, poly(ether, ketones) (PEK), polyimides (nylons), polyolefins, polystyrene, polysulfones, polyurethanes, polyvinyl chloride (PVC), silicone rubbers, polyethylene, polyurethane, latex, polyesters, poly(ethylene-terephthalate), and blends of these polymers.

7. The method of claim 1 wherein the polymeric material comprises a polymer selected from the group consisting of: poly(amino acids), polyanhydrides, polycaprolactones, poly(lacti-glycolic acid), polyhydroxybutyrates, polyorthoesters, and blends of these polymers.

8. The method of claim 1 wherein the organic dye is selected from the group consisting of: methylene blue, toluidine blue, methylene violet, azure A, azure B, azure C, brilliant cresol blue, thionin, methylene green, bromcresol green, gentian violet, acridine orange, brilliant green, acridine yellow, quinacrine, trypan blue, trypan red and mixtures of these dyes.

9. The method of claim 1 wherein the reducing agent is selected from the group consisting of: ascorbic acid, ferrous gluconate, other reducing agents and mixtures thereof.

10. The method according to claim 1 wherein the liquid composition includes a buffer to maintain a pH of between 4 and 9.

11. The method according to claim 1 wherein the buffer is citrate.

## Patentansprüche

1. Verfahren zum Herstellen von polymerem Material für ein medizinisches Gerät, wobei das genannte Verfahren Folgendes umfasst:
Inkontaktbringen des polymeren Materials mit einer flüssigen Zusammensetzung, die einen organischen Farbstoff und ein Reduktionsmittel umfasst, für eine zum Imprägnieren des polymeren Materials mit dem organischen Farbstoff ausreichende Dauer und
Entfernen des imprägnierten Polymers aus der flüssigen Zusammensetzung.

2. Verfahren nach Anspruch 1, bei dem das genannte Inkontaktbringen das Eintauchen des polymeren Materials in die flüssige Zusammensetzung für eine ausgewählte Dauer zwischen einer Minute und etwa 24 Stunden umfasst.

3. Verfahren nach Anspruch 2, bei dem das genannte Inkontaktbringen das Eintauchen des polymeren Materials in die flüssige Zusammensetzung für eine ausgewählte Dauer zwischen etwa 60 Minuten und etwa 240 Minuten umfasst.

4. Verfahren nach Anspruch 1, bei dem die flüssige Zusammensetzung eine wässrige Zusammensetzung ist.

5. Verfahren nach Anspruch 1, bei dem die flüssige Zusammensetzung ein Lösungsmittel umfasst, das aus der Gruppe bestehend aus Wasser, einem Alkohol, Tetrahydrofuran, Azeton und Gemischen davon ausgewählt ist.

6. Verfahren nach Anspruch 1, bei dem das polymere Material ein Polymer umfasst, das aus der aus Folgenden bestehenden Gruppe ausgewählt ist: Acryle, Polyacrylate, Polymethacrylate, Fluorkohlenstoffe, Hydrogele, Polyacetale, Polyamide, Polyurethan/Polycarbonate, Polyester, Poly(cther, Ketone) (PEK), Polyimide (Nylon), Polyolefine, Polystyrole, Polysulfone, Polyurethane, Polyvinylchlorid (PVC), Silikonkautschuke, Polyethylen, Polyurethan, Latex, Polyester, Poly(ethylen-tercphthalat) und Mischungen dieser Polymere.

7. Verfahren nach Anspruch 1, bei dem das polymere Material ein Polymer umfasst, das aus der aus Folgenden bestehenden Gruppe ausgewählt ist Poly(aminosäuren), Polyanhydride, Polycaprolactone, Poly(lactid-co-Glycolid), Polyhydroxybutyrate, Polyorthoester und Mischungen dieser Polymere.

8. Verfahren nach Anspruch 1, bei dem der organische Farbstoff aus der aus Folgenden bestehenden Gruppe ausgewählt ist: Methylenblau, Toluidinblau, Methylenviolett, Azur A, Azur B, Azur C. Brillantkresolblau, Thionin, Methylengrün, Bromkresolgrün, Gentianaviolett, Acrydinorange, Brillantgrün, Acridingelb, Quinacrin, Trypanblau, Trypanrot und Gemische dieser Farbstoffe.

9. Verfahren nach Anspruch 1, bei dem das Reduktionsmittel aus der Gruppe bestehend aus Ascorbinsäure, Eisengluconat, sonstigen Reduktionsmitteln und Gemischen davon ausgewählt ist.

10. Verfahren nach Anspruch 1, bei dem die flüssige Zusammensetzung einen Puffer zum Halten des pH-Wertes zwischen 4 und 9 aufweist.

11. Verfahren nach Anspruch 1, bei dem der Puffer ein Zitrat ist.

## Revendications

1. Procédé de fabrication d'un matériau polymère pour un dispositif médical, ledit procédé comprenant les étapes ci-desssous:
mise en contact du matériau polymère avec une composition liquide comprenant un colorant organique et un agent réducteur pendant une période de temps suffisante pour imprégner le matériau polymère du colorant organique ; et
retrait du polymère imprégné de la composition liquide.

2. Procédé selon la revendication 1, dans lequel ladite étape de mise en contact comprend l'étape d'immersion du matériau polymère dans la composition liquide pendant une période de temps sélectionnée de sorte à être comprise entre une minute et environ 24 heures.

3. Procédé selon la revendication 2, dans lequel ladite étape de mise en contact comprend l'étape d'immersion du matériau polymère dans la composition liquide pendant une période de temps sélectionnée de sorte à être comprise entre environ 60 minutes et environ 240 minutes.

4. Procédé selon la revendication 1, dans lequel la composition liquide est constituée par une composition aqueuse.

5. Procédé selon la revendication 1, dans lequel la composition liquide comprend un solvant sélectionné dans le groupe constitué d'eau, d'alcool, de tétrahydrofurane, d'acétone et de mélanges de ces substances.

6. Procédé selon la revendication 1, dans lequel le matériau polymère comprend un polymère sélectionné dans le groupe constitué d'acryliques, de polyacrylates, polyméthacrylates, fluorocarbures, d'hydrogels, de polyacétals, polyamides, polyuréthane/polycarbonates, polyesters, poly(éther, cétones) (PEK), polyimides (nylons), polyoléfines, polystyrène, polysulfones, polyuréthanes, chlorure de polyvinyle (PVC), caoutchoucs de silicone, polyéthylène, polyuréthane, latex, polyesters, poly(éthylène-téréphtalate) et de mélanges de ces polymères.

7. Procédé selon la revendication 1, dans lequel le matériau polymère comprend un polymère sélectionné dans le groupe constitué de : poly(aminoacides), polyanhydrides, polycaprolactones, poly(acide lactique glycolique), polyhydroxybutyrates, polyorthoesters et de mélanges de ces polymères.

8. Procédé selon la revendication 1, dans lequel le colorant organique est sélectionné dans le groupe constitué de bleu de méthylène, bleu de toluidine, violet de méthylène, d'azure A, d'azure B, d'azure C, de bleu brillant de crésol, thionine, vert de méthylène, vert de bromocrésol, violet de gentiane, d'orange d'acridine, de vert brillant, jaune d'acridine, vert brillant, jaune d'acridine, quinacrine, bleu de trypane, rouge de trypane et de mélanges de ces colorants.

9. Procédé selon la revendication 1, dans lequel l'agent réducteur est sélectionné dans le groupe constitué d'acide ascorbique, de gluconate ferreux, d'autres agents réducteurs et de mélanges de ceux-ci.

10. Procédé selon la revendication 1, dans lequel la composition liquide englobe un tampon pour maintenir un pH compris entre 4 et 9.

11. Procédé selon la revendication 1, dans lequel le tampon est constitué par de la citrate.
